(19) Europäisches Patentamt
European Patent Office
Office européen des brevets

(11) Veröffentlichungsnummer : **0 194 580**
**B1**

(12) # EUROPÄISCHE PATENTSCHRIFT

(45) Veröffentlichungstag der Patentschrift : **24.01.90**

(51) Int. Cl.$^5$ : **C 07 D233/56**, A 61 K 31/415

(21) Anmeldenummer : **86102933.8**

(22) Anmeldetag : **05.03.86**

(54) **Neue Imidazolylverbindungen und Verfahren zu ihrer Herstellung.**

(30) Priorität : **13.03.85 DE 3508905**

(43) Veröffentlichungstag der Anmeldung : **17.09.86 Patentblatt 86/38**

(45) Bekanntmachung des Hinweises auf die Patenterteilung : **24.01.90 Patentblatt 90/04**

(84) Benannte Vertragsstaaten : **AT BE CH DE FR GB IT LI LU NL SE**

(56) Entgegenhaltungen :
**EP--A-- 0 003 560**
**EP--A-- 0 015 002**
**EP--A-- 0 073 663**
**EP--A-- 0 135 177**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Lau, Hans-Hermann, Dr.**
**Kastanienhain 29**
**D-6232 Bad Soden am Taunus (DE)**
Erfinder : **Bartmann, Wilhelm, Dr.**
**Am Dachsbau 5**
**D-6232 Bad Soden am Taunus (DE)**
Erfinder : **Beck, Gerhard, Dr.**
**Gustav-Freytag-Strasse 24**
**D-6000 Frankfurt am Main 1 (DE)**
Erfinder : **Wess, Günther, Dr.**
**Hainstrasse 35**
**D-6455 Erlensee (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Imidazol und seine 1-substituierten Derivate hemmen die Thromboxan-Synthetase (H.-H. Tai, Biochem. and Biophys. Res. Comm. 80, 236 (1978)).

Das Enzym Thromboxan-Synthetase katalysiert innerhalb des Arachidonsäuremetablismus die Umwandlung der Prostaglandinendoperoxide ($PGH_2$ bzw. $PGG_2$) in das biologisch hochaktive Thromboxan $A_2$ ($TXA_2$), das die Aggregation der Blutplättchen induziert und auf die glatte Muskulatur stark konstriktiv wirkt. $TXA_2$ spielt eine wesentliche Rolle bei der Haemostase, bei pathologischen Situationen mit erhöhter Tendenz zu Gefäßkrämpfen und bzw. oder Thrombose. Weiterhin wirkt $TXA_2$ in vitro und in vivo stark kontrahierend auf die Bronchialmuskulatur (B. Samuelson, Angew. Chem., 95, 854 (1983)).

Die in der vorliegenden Erfindung beschriebenen, neuen 1-Imidazolylalkylnaphthoesäuren und deren Säurederivate zeichnen sich durch eine spezifische Hemmwirkung auf das Enzym Thromboxan-Synthetase aus.

Die Verbindungen eignen sich daher zur Prophylaxe oder zur Behandlung von Krankheiten mit gestörter (erhöhter) Thrombozytenaggregationsneigung, sowie bei pathologisch erhöhten Thromboxanspiegeln, die bei Ischämie, Angina pectorics, thromboembolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma und Apnoe, Entzündungskrankheiten sowie mikrovaskulären Komplikationen bei Diabetes mellitus gefunden werden. Die erfindungsgemäßen Verbindungen beeinflussen günstig Erkrankungen mit erhöhten Thromboxanspiegeln in verschiedenen Organen, beispielsweise im Bereich der Nieren oder im Magen-Darm-Trakt bei Collitis oder bei « inflammatory bowel disease ». Zudem sind die Verbindungen geeignet, die Proliferation von Tumorzellen zu verlangsamen oder zu verhindern. EP-A-0003560 betrifft Imidazolderivate, die in 1-Stellung mit einer Alkylnaphtylgruppe substituiert sind ; die Naphtylgruppe ist niemals durch eine COOH oder COO-Alkylgruppe substituiert. Die Imidazolderivate gemäß EP-A-0015002 sind in 1-Stellung mit einem nicht-aromatischen Rest substituiert. Schließlich betrifft EP-A2-0073663 u. a. 1-Imidazolylalkylnaphtholsäurederivate, welche ebenfalls pharmakologisch wirksam sind.

Gegenstand der vorliegenden Erfindung sind neue 6-(1-Imidazolylalkyl)-2-naphthoesäuren und deren Derivate der allgemeinen Formel I,

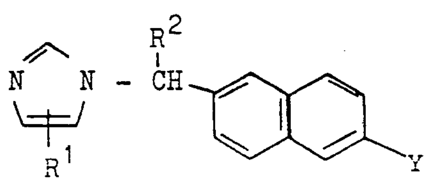

I

sowie die physiologisch verträglichen Säureadditionssalze. In der allgemeinen Formel I bedeuten :

$R^1$ Wasserstoff, oder in 2-, 4- oder 5-Position einen $C_1$-$C_4$-Alkylrest

$R^2$ Wasserstoff oder einen $C_1$-$C_4$-Alkylrest

Y einen Rest der Formel $—CO_2R^3$, wobei

$R^3$ Wasserstoff, $C_1$-$C_4$-Alkyl oder ein physiologisch verträgliches Metall-, $NH_4$- oder ein Ammoniumion, das sich von einem primären, sekundären oder tertiären Amin ableitet, oder ein Tetraalkylammoniumion bedeutet,

mit Ausnahme der Verbindungen der Formel I, worin $R^1$ und $R^2$ gleichzeitig Wasserstoff sind. Von ganz besonderer Bedeutung sind Verbindungen der Formel I, worin $R^1$ Wasserstoff oder $C_1$-$C_4$-Alkyl in 5-Stellung, $R^2$ Wasserstoff oder $C_1$-$C_2$-Alkyl und Y die COOH- oder COO-$R^3$-Gruppe, wobei $R^3$ $C_1$-$C_4$-Alkyl bedeutet, darstellen sowie die physiologisch verträglichen Säureadditionssalze.

Ferner umfaßt die Erfindung Säureadditionssalze der beschriebenen Verbindungen mit anorganischen oder organischen Säuren, z. B. Chlorwasserstoffsäure, Bromwasserstoffsäure, Schwefelsäure, Phosphorsäure, Salpetersäure, Essigsäure, Propionsäure, Oxalsäure, Malonsäure, Glykolsäure, Bernsteinsäure, Maleinsäure, Fumarsäure, Weinsäure, Citronensäure, Benzoesäure oder Zimtsäure.

Gegenstand der Erfindung ist ferner ein Verfahren zur Herstellung von Verbindungen der Formel I, das dadurch gekennzeichnet ist, daß man

a) eine Verbindung der Formel II

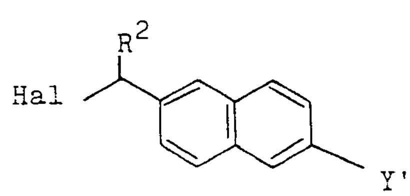

II

worin $R^2$ die zur Formel I angegebene Bedeutung hat, Y' die Reste

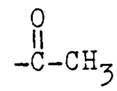

oder $-COOR^7$ bedeutet, wobei $R^7$ $C_1$-$C_4$-Alkyl darstellt, und Hal Chlor oder Brom bedeutet, umsetzt mit einem Imidazol der Formel III

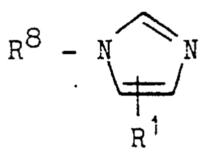

III

worin $R^1$ die zur Formel I angegebene Bedeutung hat und $R^8$ Wasserstoff, niederes Alkanoyl oder Benzoyl bedeutet, zu einer Verbindung der Formel IV

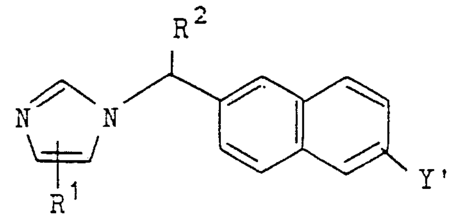

IV

worin $R^1$ und $R^2$ die zur Formel I und Y' die zur Formel II angegebene Bedeutungen haben,

b) gegebenenfalls eine Verbindung der Formel IV, worin Y' den Rest

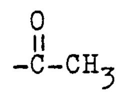

darstellt, oxidiert zu einer Verbindung der Formel I worin Y den Rest —COOH bedeutet,

c) gegebenenfalls eine Verbindung der Formel I worin Y den Rest $-COOR^3$ darstellt, wobei $R^3$ die zur Formel I genannten Bedeutungen hat, jedoch nicht Wasserstoff oder ein Kation darstellt, hydrolysiert zu einer Verbindung der Formel I, worin Y den Rest —COOH darstellt,

d) gegebenenfalls eine Verbindung der Formel I worin Y den Rest —COOH darstellt, in die entsprechenden Ester oder Salze der Formel I, worin Y den Rest $-COOR^3$ mit $R^3 \neq$ Wasserstoff bedeutet, überführt und

e) gegebenenfalls eine erhaltene Verbindung der Formel I in die Säureadditionssalze überführt.

Es können eine oder mehrere der Gegebenenfallsmaßnahmen durchgeführt werden ; die Reihenfolge der Maßnahmen c) und d) ist beliebig.

Die Ausgangsverbindungen der Formel II sind entweder bekannt oder können nach in der Literatur beschriebenen Methoden erhalten werden. So erhält man sie z. B. durch Halogenierung einer Verbindung der Formel V

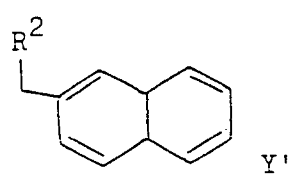

V

worin $R^2$ die zu Formel I und Y' die zu Formel II angegebenen Bedeutungen hat.

2-Acetylnaphthalinderivate der allgemeinen Formel V können nach bekannten Verfahren (z. B. J. Org. Chem., 49, 384 (1984) bzw. Eur. J. Med. Chem.-Chim. Ther., 19, 5 (1984)) hergestellt werden.

Verbindungen der Formel II mit $Y' = CO_2R^7$ können u. a. nach dem in DOS 23 63 416 beschriebenen Verfahren erhalten werden.

Imidazolderivate der Formel III mit $R^8$ = Acyl können nach literaturbekannten Verfahren (z. B. J. Amer. Chem. Soc., 74, 6274 (1952) oder J. Org. Chem., 45, 4038 (1980)) hergestellt werden.

Verbindungen der Formel I mit $Y = CO_2R^3$, worin $R^3$ Alkyl bedeutet, und Verbindungen der Formel IV werden zweckmäßigerweise erhalten durch Umsetzung von Imidazolderivaten der Formel III mit $R^8$ = Wasserstoff mit Verbindungen der Formel II in Gegenwart von geeigneten starken Basen wie z. B. Natriumhydrid, Natriumhydroxid, Natriumalkoholat, Kalium-t-butylat oder Kaliumcarbonat in einem inerten Lösungsmittel wie Aether, Tetrahydrofuran, Dimethoxyethan, Dioxan, Toluol, Xylol, Aceton, Formamiden, Dimethylsulfoxid oder Alkoholen zwischen 0° und 180 °C.

Verbindungen der Formel I mit $Y = CO_2R^3$ mit $R^3$ = Alkyl, bzw. Verbindungen der Formel IV werden ebenfalls erhalten, wenn man Imidazolderivate der Formel III mit $R^8$ = Acyl mit Verbindungen der Formel III in einem geeigneten Lösungsmittel, insbesondere Acetonitril, bei Temperaturen zwischen 0° und 80 °C umsetzt. (s. Chem. Pharm. Bull., 30, 4242 (1982)).

Die Imidazolketone der Formel IV können in einer Haloform-Reaktion mit Chlor, Brom oder Jod in Gegenwart einer starken Base wie z. B. Natrium- oder Kaliumhydroxyd zu Verbindungen der Formel I oxidiert werden, in denen Y eine Carboxylgruppe bedeutet.

Nach literaturbekannten Verfahren lassen sich aus Verbindungen der Formel I mit Y = Carboxyl die entsprechenden Ester bzw. Salze herstellen.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation oder Säulen-, Dünnschicht- oder Hochdruckflüssigkeitschromatographie.

Die Verbindungen der allg. Formel I zeigen eine spezifische Hemmung der Thromboxan-Synthese und können deshalb als Arzneimittel zur Prophylaxe oder Behandlung von Krankheiten mit gestörter, d. h. erhöhter Aggregationsneigung der Thrombocyten, sowie bei pathologisch erhöhten Thromoxanspiegeln, wie sie bei Ischämie, Angina pectoris, thrombo-embolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma oder Apnoe, Entzündungskrankheiten sowie mikrovaskulären Komplikationen bei der Diabetes mellitus gefunden werden, verwendet werden. Die erfindungsgemäßen Verbindungen beeinflussen Erkrankungen mit erhöhten Thromboxanspiegeln in verschiedenen Organen günstig, z. B. im Nieren- oder im Magen-Darm-Bereich bei Colitis oder bei « inflammatory bowel disease ». Die Verbindungen sind zudem geeignet, die Proliferation von Tumorzellen zu verlangsamen oder sogar zu verhindern.

Die Metaboliten der Arachidonsäure sind an einer Reihe von physiologischen und pathophysiologischen Prozessen beteiligt. Bei der Regulation des Tonus der Blutgefäße und der Thrombocytenaggregation sind Prostacyclin ($PGI_2$) und Thromboxan $A_2$($TXA_2$) von wesentlicher Bedeutung. Prostacyclin, das bevorzugt in den Endothelzellen der Blutgefäße aus Prostaglandinendoperoxid $H_2$($PGH_2$) gebildet wird, bewirkt eine Vasodilation und verhindert gleichzeitig die Aggregation der Thrombocyten. Die Umwandlung von $PGH_2$ in Prostacyclin wird durch die Prostacyclin-Synthetase katalysiert. Der physiologische Gegenspieler von Prostacyclin ist Thromboxan $A_2$, das vornehmlich in den Blutplättchen aus $PGH_2$ synthetisiert wird. Diese Reaktion wird durch das Enzym Thromboxan-Synthetase katalysiert. $TXA_2$ bewirkt eine Aggregation der Blutblättchen und führt zu einer Vasokonstriktion. Es ist bislang der stärkste bekannte Vasokonstriktor im menschlichen Organismus (s. A. G. Herman, P. M. Vonhoutte, H. Denolin, A. Goossens, Cardiovascular Pharmacology of the Prostaglandins, Raven Press, New York, 1982). Störungen im Gleichgewicht zwischen Prostacyclin und Thromboxan $A_2$ führen zu pathophysiologischen Situationen. Bei gleichbleibenden $PGI_2$-Spiegeln führt eine Erhöhung des Thromboxan-Spiegels daher zu einer Blutplättchenaggregation und zu Gefäßspasmen sowie zu einer erhöhten Anfälligkeit gegenüber Atherothrombose (Lancet 1977, 479 ; Science 1976, 1135 ; Amer. J. Cardiology 41, 787 (1978) ; Lancet 1977, 1216). Bei der experimentellen Atherosklerose ist die Bildung von $PGI_2$ inhibiert bei gleichzeitig erhöhter Thromboxan $A_2$-Bildung (Prostaglandins 14, 1025 und 1035 (1977)). Aus diesem Grunde wird $TXA_2$ mit verschiedenen Anginen, myocardialer Infarktbildung, plötzlichem Herztod und Schlaganfällen in Verbindung gebracht (Thromb. Haemostasis 38, 132 (1977)) ; Platelets, Prostaglandins and Cardiovascular System, Florenz, Februar 1984).

Ein weiteres Gebiet, auf dem eine Störung des $PGI_2/TXA_2$-Gleichgewichtes als ein beitragender Faktor angesehen wird, ist die Migräne. Der Migränekopfschmerz ist mit Veränderungen des intra- und extracerebralen Blutflusses verbunden, insbesondere mit einer vor dem Auftreten des Kopfschmerzes erfolgenden Reduzierung des cerebralen Blutflusses und nachfolgender Dilatation in beiden Gefäßbereichen während der Kopfschmerzphase. Thrombocyten von Migränepatienten besitzen eine größere Neigung zur Aggregation als diejenigen von normalen Individuen (J. clin. Pathol. 24, 250 (1971) ; J. Headache, 17, 101 (1977) ; Lancet 1978, 501).

Bei Patienten mit Diabetes mellitus wird ein Ungleichgewicht zwischen Prostacyclin und Thromboxan $A_2$ als Ursache für die mikrovaskulären Komplikationen angesehen. Thrombocyten von Diabetespatienten bilden erhöhte Mengen an $TXA_2$ und Malondialdehyd (Symposium « Diabetes and Thrombosis-Implications für Therapy », Leeds, Großbritannien, April 1979). Es wurde außerdem gezeigt, daß bei Ratten mit experimentell erzeugter Diabetes die vaskuläre $PGI_2$-Bildung gehemmt ist, während die $TXA_2$-Synthese in den Plättchen erhöht ist (IV. Int. Prostaglandin Conference, Washington DC, Mai 1979).

Nichtsteroidale Antiinflammatorika hemmen die Cyclooxygenase, die die Umwandlung der Arachidonsäure in $PGH_2$ über $PGG_2$ katalysiert. Sie greifen daher sowohl in die Biosynthese des Thromboxan $A_2$ als auch in die des Prostacyclins ein. Wertvoller wären somit Verbindungen, die spezifisch die Bildung

von Thromboxan $A_2$ durch Hemmung der Thromboxan-Synthetase blockieren und gleichzeitig die Bildung von Prostacyclin nicht beeinflussen.

Versuchsbericht

Die biochemische und pharmakologische Aktivität wurde in folgenden Testsystemen bestimmt :

1. Hemmung der Arachidonsäure-induzierten Aggregation von Humanthrombocyten in vitro

Gesund erscheinenden männlichen und weiblichen Freiwilligen, die im vorangegangenen Zeitraum von 10 Tagen keine Medikamente eingenommen haben, wird durch vorsichtige Kanülierung der Antekubitalvene Blut entnommen, das sofort mit Natriumcitrat (a d 0.38 %) stabilisiert wird. Durch 15 minütiges Zentrifugieren bei 140 × g erhält man im Überstand plättchenreiches Plasma (PRP), dessen Thrombocytengehalt im Bereich 2.5 — 3.5 × $10^8$/ml (Coulter Counter) liegen sollte. Die Plättchenaggregation wird optisch durch Messung der Lichttransmission in einem Born'schen Aggregometer verfolgt. Das Gesamtvolumen des Testansatzes beträgt 0.25 ml. Die Vorinkubationszeit mit Prüfpräparat beträgt 10 min bei 37 °C, die Aggregationsinduktion erfolgt danach mit 2 × $10^{-4}$ M Arachidonsäure. Das Prüfpräparat wird in der Regel in fünf verschiedenen Konzentrationen in PRP aus drei verschiedenen Spendern getestet. Aus den jeweiligen maximalen Aggregationsamplituden werden Dosis-Wirkungskurven erstellt und graphisch $IC_{50}$-Werte* bestimmt. Die Messungen erfolgen im Zeitraum von 1-6 Stunden nach der Blutentnahme.

Von den erfindungsgemäßen Verbindungen wurden nach der oben beschriebenen Methode folgende $IC_{50}$-Werte für die Hemmung der Arachidonsäure-induzierten Aggregation von Humanthrombocyten in vitro ermittelt :

| Beispiel | $IC_{50}$-Wert (Mol/l) |
|---|---|
| 2 | $3 \times 10^{-6}$ |
| 4 | $5.4 \times 10^{-7}$ |

* ($IC_{50}$ ist die Konzentration, die eine 50 %ige Hemmung der Arachidonsäure-induzierten Aggregation bewirkt).

2. Thrombin induzierte $TXA_2$-Freisetzung in plättchenreichem Humanplasma in vitro

Gesund erscheinenden männlichen und weiblichen Freiwilligen, die im vorangegangenen Zeitraum von 10 Tagen keine Medikamente eingenommen haben, wird durch vorsichtige Kanülierung der Antekubitalvene Blut entnommen, das sofort mit Natriumzitrat (a d. 0,38 %) stabilisiert wird. Durch 15 minütiges Zentrifugieren bei 140 × g erhält man im Überstand plättchenreiches Plasma (PRP), dessen Thrombozytengehalt im Bereich 2,5 — 3,5 · $10^8$/ml liegen sollte. Durch erneutes Zentrifugieren (10 min bei 2 000 × g) werden die Thrombozyten sedimentiert und anschließend in Tyrode-Lösung re-solubilisiert (ca. 7 · $10^7$ Plättchen/ml, Gesamtvolumen pro Messung 0,5 ml). Nach Zugabe von Testsubstanz wird 10 min bei 37 °C inkubiert, anschließend 7,2 · $10^{-7}$ M Arachidonsäure und 0,5 E Thrombin zugesetzt und 30 min bei 37 °C inkubiert. Hierauf wird im Eisbad abgestoppt und nach Zugabe von Tracer und $TXB_2$-spezifischem Antikörper (NEN, Dreieich) der $TXB_2$-Gehalt radioimmunologisch bestimmt ($TXA_2$ ist unter den Versuchsbedingungen instabil und daher nicht meßbar. Statt dessen wird das stabile Hydrolyseprodukt $TXB_2$ gemessen). Meßgröße ist der relative $TXB_2$-Gehalt in den Thrombozyten-Inkubationen aus zwei bis drei verschiedenen Spendern mit und ohne (= 100 %) Prüfsubstanz.

Nach der oben beschriebenen Methode wurden von den erfindungsgemäßen Verbindungen z. B. folgende Werte für die $TXB_2$-Freisetzung nach Gabe von Testsubstanz ermittelt :

| Beispiel | $TXB_2$-Freisetzung |
|---|---|
| 2 | $10^{-6}$ Mol/l : 31 % |
| | $10^{-7}$ Mol/l : 55 % |
| 4 | $10^{-6}$ Mol/l : 9 % |
| 4 | $10^{-7}$ Mol/l : 19 % |
| | $10^{-8}$ Mol/l : 98 % |

3. Hemmung der Laser-induzierten Thrombose

Die Untersuchungen der erfindungsgemäßen Verbindungen im Laser-induzierten Thrombosemodell erfolgt an männlichen oder weiblichen Sprague-Dawley-Ratten mit einem Körpergewicht von ca 200 g. Die zu untersuchenden Tiere werden mit 0.1 mg Atropinum sulf. sol. s. c. prämediziert und mit 100 mg Ketaminhydrochlorid und 4 mg Xylazin pro kg Körpergewicht i. p. anästhetisiert. Zur Untersuchung dienen Arteriolen des Mesenteriums mit einem Durchmesser von 12-25 µm. Während der Messung erfolgt eine Hyperfundierung des exponierten Mesenteriums mit erwärmter phys. NaCl (37 °C) oder eine Überschichtung mit entgastem Paraffinöl.

Der Strahl des 4 W Argon-Lasers (Fa. Spectra Physics, Darmstadt, FRG) wird mittels einer Strahladaptions- und -justieranlage (Fa. BTG, München, FRG) koaxial in den invertierten Strahlengang eines Mikroskopes (ICM 405, LD-Epilan 40/0.60 ; Fa. Zeiss, Oberkochen, FRG) eingebracht. Die benutzte Wellenlänge beträgt 514.5 nm mit einer Energie oberhalb des Objektives von 40 mW. Die Expositionszeit pro Einzelschuß dauert 1/15 sec. Der Durchmesser des effektiven Laserstrahls auf dem Gefäß beträgt 10 μm, bei wiederholter Exposition erfolgt der nächste Schuß jeweils 5 μm stromaufwärts unmittelbar an der Gefäßwand. Alle Meßvorgänge werden per Videokamera (Sony, Trinicon-Röhre) aufgenommen und auf einem Recorder (Sony, U-matic 3/4) gespeichert. Im Durchlichtverfahren des gleichen Mikroskops wird bei kleiner Vergrößerung (LD-Epilan 8/0.20) ein Überblicksbild der zu untersuchenden Endstrombahn geliefert. Ein Videoanalyser sowie ein Korrelator dient zur Bestimmung der Strömungsgeschwindigkeit in der untersuchten Arteriole.

Die Testsubstanzen wurden in 0.9 % Natriumchlorid-Lösung (enthielt 1 % Carboxymethylzellulose oder in entsprechenden Lösungsvermittlern) den Versuchstieren in verschiedenen Dosierungen oral eine Stunde vor Versuchsbeginn verabreicht ; Kontrolltiere wurden in entsprechender Weise, aber ohne Testsubstanzen, behandelt. Die Untersuchungen wurden randomisierend als Doppelblindstudie durchgeführt.

Auswertung

Es wurde die Anzahl der Schüsse gezählt, um einen definierten Thrombus zu induzieren. Die Frequenz des Laserblitze betrug eine Läsion alle 2 Minuten, wobei alle während des Beobachtungszeitraumes gebildeten Thromben von einer Mindestgröße von 1/4 Gefäßradius ausgezählt und vermessen wurden. Die statistische Auswertung der Versuchsergebnisse erfolgte mit Hilfe des $\chi^2$-Testes (L. Cavalli-Sforza, Biometrie, Stuttgart, 1969, S 49 f.).

| Beispiel | Zahl der Laserschüsse zur Bildung eines Thrombus (Vergl. z. Kontrolle) |
|---|---|
| | 10 mg/kg p. o. + 96 % |
| 4 | 5 mg/kg p. o. + 53 % |
| | 1 mg/kg p. o. + 16 % |

Die Verbindungen der Formel I blockieren spezifisch die Bildung von Thromboxan $A_2$ durch Hemmung der Thromboxan-Synthetase ohne Beeinflussung der Prostacyclinbildung und eignen sich daher zur Vorbeugung oder zur Behandlung der oben aufgeführten Erkrankungen, die auf eine Hemmung der Thromboxan-Synthetase ansprechen.

Die Erfindung betrifft daher ferner pharmazeutische Präparate auf Basis der erfindungsgemäßen Verbindungen. Die Verbindungen der Formel I werden in verschiedenen Dosierungsformen verabreicht, z. B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die erfindungsgemäßen Verbindungen der Formel I können als freie Basen oder in Form ihrer physiologisch unbedenklichen anorganischen oder organischen Säureadditionssalze zur Anwendung kommen. Die freien Basen und Säureadditionssalze können in Form ihrer wässrigen Lösungen oder Suspensionen oder auch gelöst oder suspendiert in pharmakologisch unbedenklichen organischen Lösungsmitteln wie ein- oder mehrwertigen Alkoholen wie z. B. Ethanol, Ethylenglykol oder Glycerin, in Triacetin, in Alkohol-Acetaldehyd-diacetalgemischen, Ölen wie z. B. Sonnenblumenöl oder Lebertran, Ethern wie z. B. Diethylenglykoldimethylether oder auch Polyethern wie z. B. Polyethylenglykol oder auch in Gegenwart anderer pharmakologisch unbedenklicher Polymerträger wie z. B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen Suppositorien oder Aerosole in Frage kommen.

Die Verbindungen sind in Dosen von 0,01 mg/kg bis 10 mg/kg wirksam. Die verabreichte Einzeldosis kann zwischen 1 mg bis 500 mg liegen. Bei oraler Applikation liegt die bevorzugte Tagesdosis zwischen 1 mg und 1 g.

Beispiel 1

6-(5-Methylimidazol-1-ylmethyl)-2-naphthoesäuremethylester

α) 1-Acetyl-4-methylimidazol

50 g (0,6 Mol) 4-Methylimidazol werden in 500 ml abs. Ether gelöst. Nach Zutropfen von 23,6 g (0,3 Mol) Acetylchlorid in 75 ml abs. Ether bei Raumtemperatur wird 11 Stunden unter Rückfluß erhitzt. Es wird vom Rückstand abfiltriert und die Lösung in Vakuum eingeengt. Erhalten werden 11,45 g (31 %) 1-Acetyl-4-methylimidazol als farbloses Öl.

$^1$H-NMR ($CDCl_3$, 60 MHz) δ = 2,15 und 2,5 (2s, 6H ; 2 × $CH_3$), 7,05 (s, 1H ; C-5-H), 7,95 (s, 1H ; C-2-H).

β) 6-(5-Methylimidazol-1-ylmethyl)-2-naphthoesäuremethylester

2 g (7,16 mMol) 6-Brommethyl-2-naphthoesäuremethylester und 1,07 g (8,6 mMol) 1-Acetyl-4-methylimidazol werden in abs. Acetonitril unter Rückfluß erhitzt (8 Stunden). Nach Einengen der Reaktionslösung wird der Rückstand in 2 N-HCl aufgenommen und mit Essigester extrahiert. Die Essigesterphasen werden verworfen. Nach Neutralisieren der wäßrigen Phase mit Natriumcarbonat wird mit Essigester extrahiert, die Essigesterphase getrocknet und im Vakuum eingeengt. Durch Verreiben des Rückstandes mit Ether werden 1,4 g (70 %) 6-(5-Methylimidazol-1-ylmethyl)-2-naphthoesäuremethylester erhalten. Schmp. 101-2 °C.

$^1$H-NMR ($CDCl_3$, 60 MHz) δ = 2,1 (d, J = 1,5 Hz, 3H ; C-$CH_3$), 3,95 (s, 3H ; $OCH_3$), 5,2 (s, 2H ; $CH_2$), 6,85-8,55 (m, 8H ; arom. H).

Beispiel 2

6-[1-(Imidazol-1-yl) ethyl]-2-naphthoesäure-Hydrochlorid

α) 2-Acetyl-6-ethylnaphthalin

100 g (0,75 Mol) Aluminiumchlorid werden in 200 ml dest. Nitrobenzol gelöst. Bei 70 °C wird eine Lösung von 100 g (0,64 Mol) 2-Ethylnaphthalin und 52 ml (57,4 g, 0,73 Mol) Acetylchlorid in 400 ml dest. Nitrobenzol zugetropft und vier Stunden nachgerührt. Die Reaktionsmischung wird auf Eis gegeben und mit 300 ml 2 N HCl angesäuert, über Klärschicht filtriert und die Phasen getrennt.

Die organische Phase wird mit 2 N HCl, Natriumcarbonat-Lösung und schließlich mit Wasser gewaschen ; die wäßrigen Phasen werden mit Dichlormethan reextrahiert. Nach Trocknen der organischen Phasen wird im Vakuum destilliert. Erhalten werden 80,3 g (63 %) eines 5 : 2-Gemisches aus 2-Acetyl-6-ethylnaphthalin und 1-Acetyl-7-ethylnaphthalin. Sdp. 0,2 : 125 °C.

Zur Trennung der Isomeren wird das Gemisch mit 44,3 ml (49,6 g, 0,8 Mol) Ethylenglykol und 0,5 g p-Toluolsulfonsäure in 800 ml Toluol am Wasserabscheider unter Rückfluß erhitzt, bis kein Wasser mehr abgeschieden wird, und somit in die isomeren Ethylenacetale überführt. Die Reaktionslösung wird mit Natriumbicarbonatlösung gewaschen und anschließend im Vakuum eingeengt. Erhalten werden 97,4 g (102 %) eines Gemisches aus zwei isomeren Ethylenacetalen, von dem 40 g durch Chromatographie mit Cyclohexan-Essigester-Gemischen über Kieselgel in die Isomeren getrennt wird. Erhalten werden 11,5 g (47,5 mMol) 7-Ethyl-3-naphthylmethylketonethylenacetal als Öl, $R_F$ (Cyclohexan-Essigester 7 : 1) : 0,38 und 25,5 g eines Öls, aus dem 16,1 g (31 %) 6-Ethyl-2-naphthylmethyl-ketonethylenacetal durch Umkristallisieren aus Petrolether isoliert werden.

Schmp. 66-68 °C, $R_F$ (Cyclohexan-Essigester 7 : 1) : 0,30.

Anschließend werden 16,1 g (66,4 mMol) 6-Ethyl-2-naphthylmethylketonethylenacetal in 100 ml Tetrahydrofuran gelöst, mit 100 ml Methanol und 100 ml 2 N HCl versetzt und drei Stunden unter Rückfluß erhitzt. Nach Einengen der Lösung auf die Hälfte des Volumens wird mit ges. Natriumbicarbonatlösung neutralisiert und mit Essigester extrahiert. Die Essigesterphase wird getrocknet und im Vakuum eingeengt. Erhalten werden 12,9 g (98 %) 2-Acetyl-6-ethylnaphthalin als Öl. $R_F$ (Cyclohexan-Essigester 7 : 1) : 0,19.

$^1$H-NMR ($CDCl_3$, 60 MHz) δ = 1,3 (t, 3H ; $CH_2$—$C\underline{H}_3$), 2,65 (s, 3H ; $CH_3$—CO), 2,8 (q, 2H ; $CH_2$), 7,1-8,0 (m, 5H ; arom. H), 8,3 (s, 1H ; C—1—H).

β) 2-Acetyl-6-(1-bromethyl) naphthalin

12,8 g (64,6 mMol) 2-Acetyl-6-ethylnaphthalin, 10,92 g (61,35 mMol) N-Bromsuccinimid und 0,1 g Azo-bis-isobutyronitril werden in 280 ml abs. Tetrachlormethan eine Stunde unter Rückfluß erhitzt. Es wird vom Rückstand abfiltriert, mit heißem Tetrachlormethan nachgewaschen und die Tetrachlormethanlösung im Vakuum eingeengt. Durch Umkristallisieren des Rückstandes aus n-Hexan-Essigester werden 9,84 g (58 %) 2-Acetyl-6-(1-bromethyl) naphthalin erhalten. Schmp. 64-66 °C.

$^1$H-NMR ($CDCl_3$, 60 MHz) δ = 2,1 (d, 3H ; CH—$C\underline{H}_3$), 2,7 (s, 3H ; C(O)—$CH_3$), 5,3 (q, 1H ; CH), 7,4-8,1 (m, 5H ; arom. H), 8,3 (s, 1H ; C—1—H).

γ) 2-Acetyl-6-[1-(imidazol-1-yl) ethyl] naphthalin

9,82 g (35,4 mMol) 2-Acetyl-6-(1-bromethyl) naphthalin, 6,38 g (70,9 mMol) Natriumimidazolid (aus 3,1 g (70,9 mMol) Natriumhydrid-Dispersion (55 %) und 4,83 g (70,9 mMol) Imidazol) und 0,588 g (3,54 mMol) Kaliumjodid werden in 100 ml abs. N,N-Dimethylformamid bei 100 °C gerührt. (24 Stunden). Anschließend wird im Vakuum eingeengt, in 4 N HCl aufgenommen, mit Essigester extrahiert und die wäßrige Phase mit Natriumcarbonatlösung neutralisiert. Dann wird 4 x mit Dichlormethan extrahiert, die Dichlormethanphase getrocknet und im Vakuum eingeengt. Der Rückstand wird mit Essigester-Methanol 8 : 1 über Kieselgel filtriert. Erhalten werden 6,48 g (70 %) 2-Acetyl-6-[1-(imidazol-1-yl) ethyl] naphthalin als Öl. $R_F$ (Essigester-Methanol 8 : 1) : 0,15.

$^1$H-NMR ($CDCl_3$, 60 MHz) δ = 1,95 (d, 3H, J = 7Hz ; $CH_3$), 2,7 (s, 3H ; $CH_3$), 5,5 (q, 1H ; CH), 6,9-8,1 (m,

8H ; arom. H), 8,4 (s, 1H ; C—1—H).

δ) 6-[1-(Imidazol-1-yl) ethyl]-2-naphthoesäure-Hydrochlorid

9,72 g (0,243 Mol) Natriumhydroxid werden in 50 ml Wasser gelöst und bei 0 bis 10 °C mit 3,75 ml (11,65 g, 72,9 mMol) Brom versetzt. Bei 0 bis 10 °C wird eine Lösung von 6,4 g (24,3 mMol) 2-Acetyl-6-[1-(imidazol-1-yl) ethyl] naphthalin in 15 ml Dioxan zugetropft. Es wird 3 Stunden bei Raumtemperatur gerührt. Nach Zugabe von 40 ml ges. Natriumbisulfit-Lösung wird 30 Minuten gerührt. Es wird mit Ether extrahiert und die wäßrige Phase dann mit 2 N HCl auf PH 1 eingestellt und im Vakuum eingeengt. Der Rückstand wird mit Isopropanol ausgekocht. Erhalten werden nach Umkristallisieren aus 2 N HCl 3,12 g (43 %) 6-[1-(Imidazol-1-yl) ethyl]-2-naphthoesäure-Hydrochlorid. Schmp. 246-9 °C (Zers.).

$^1$H-NMR ($D_2O$, 60 MHz) δ = 2,0 (d, 3H ; $CH_3$), 6,0 (q, 1H ; $CH_3$), 7,1-8,8 (m, 9H ; arom. H).

Beispiel 3

6-(1-Imidazolylmethyl)-2-naphthoesäuremethylester

10 g (35,8 mMol) 6-Brommethyl-2-naphthoesäuremethylester und 9,75 g (0,143 Mol) Imidazol werden in 300 ml Dimethoxyethan 3 Stunden unter Rückfluß erhitzt. Nach Einengen der Reaktionslösung wird in 2 N HCl aufgenommen, mit Essigester extrahiert und die Essigesterphase verworfen.

Die wäßrige Phase wird mit Natriumbicarbonat auf PH 7 eingestellt und der Niederschlag abgesaugt. Unter Zusatz von wenig Aktivkohle wird der Rückstand aus Essigester umkristallisiert. Erhalten werden 7,16 g (75 %) 6-(1-Imidazolylmethyl)-2-naphthoesäuremethylester. Schmp. 144-6 °C.

IR (KBr) 1 710 $cm^{-1}$.

$^1$H-NMR ($CDCl_3$, 60 MHz) δ = 4,0 (s, 3H ; $CH_3$), 5,3 (s, 2H ; $CH_2$), 6,9-8,2 (m, 8H ; arom. H), 8,55 (s, 1H ; C—1—H).

Beispiel 4

6-(5-Methylimidazol-1-yl)methyl-2-naphthoesäure-Hydrochlorid

3,31 g (11,8 mMol) 6-(5-Methylimidazol-1-yl)methyl-2-naphthoesäuremethylester werden drei Stunden in 40 ml 2 N HCl unter Rückfluß erhitzt, mit Aktivkohle versetzt und heiß filtriert. Beim Abkühlen kristallisiert das Hydrochlorid der Säure aus. Erhalten werden nach Absaugen, Nachwaschen mit Aceton und Trocknen 3,03 g (85 %) 6-(5-Methylimidazol-1-yl)-methyl-2-naphthoesäure-Hydrochlorid. Schmp. : 289-90 °C.

$^1$H-NMR ($D_2O$, 60 MHz) δ = 2,15 (s, 3H ; $CH_3$), 5,45 (s, 2H ; $CH_2$), 7,1-8,7 (m, 8H ; arom. H).

Beispiel 5

6-[1-(Imidazol-1-yl) ethyl]-2-naphthoesäuremethylester

1,26 g (4,2 mMol) 6-[1-(Imidazol-1-yl) ethyl]-2-naphthoesäure-hydrochlorid werden in 25 ml gesättigter methanolischer HCl-Lösung eine Stunde unter Rückfluß erhitzt und dann im Vakuum eingeengt. Der Rückstand wird in gesättigter Natriumbicarbonatlsg. aufgenommen und mit Essigester extrahiert. Nach Trocknen und Einengen der Essigesterlösung wird der Rückstand über Kieselgel chromatographiert (Essigester-Methanol 8 : 1). Erhalten werden 530 mg (45 %) 6-[1-(Imidazol-1-yl)ethyl]-2-naphthoesäuremethylester. Schmp. 103-6 °C.

$^1$H-NMR ($CDCl_3$, 60 MHz) δ = 1,95 (d, 3H ; $CH_3$), 3,95 (s, 3H ; $OCH_3$), 5,5 (q, 1H ; CH), 6,9-8,6 (m, 9H ; arom. H).

**Patentansprüche** (für die Vertragsstaaten : BE, CH, DE, FR, GB, IT, LI, LU, NL, SE)

1. Verbindung der Formel I

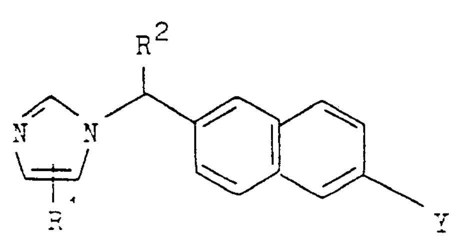

I

worin bedeuten :

$R^1$ Wasserstoff, oder in 2-, 4- oder 5-Position einen $C_1$-$C_4$-Alkylrest,

$R^2$ Wasserstoff oder einen $C_1$-$C_4$-Alkylrest

Y einen Rest der Formel $—CO_2R^3$, wobei

$R^3$ Wasserstoff, $C_1$-$C_4$-Alkyl oder ein physiologisch verträgliches Metall, $—NH_4—$ oder ein Ammoniumion, das sich von einem primären, sekundären oder tertiären Amin ableitet, oder ein Tetraalkylammoniumion bedeutet,

sowie die physiologisch verträglichen Säureadditionssalze

mit Ausnahme der Verbindungen der Formel I worin $R^1$ und $R^2$ gleichzeitig Wasserstoff sind.

2. Verbindungen gemäß Anspruch 1, dadurch gekennzeichnet, daß in Formel I $R^1$ Wasserstoff oder $C_1$-$C_4$-Alkyl in 5-Stellung, $R^2$ Wasserstoff oder $C_1$-$C_2$-Alkyl und Y die —COOH— oder $COOR^3$-Gruppe, worin $R^3$ $C_1$-$C_4$-Alkyl darstellt, bedeuten.

3. Arzneimittel, gekennzeichnet durch einen Gehalt an einer Verbindung gemäß Anspruch 1 sowie pharmazeutisch üblichen Hilfs- und Trägerstoffen.

4. Eine Verbindung gemäß Anspruch 1 zur Verwendung als Arzneimittel.

5. Verfahren zur Herstellung von Verbindungen der Formel I gemäß Anspruch 1,

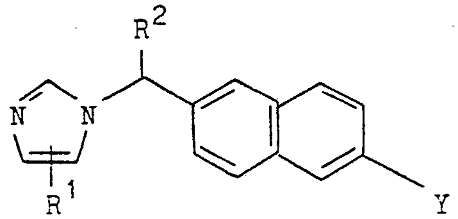

I

worin bedeuten $R^1$, $R^2$ und Y die angegebene Bedeutung haben

sowie von deren physiologisch verträglichen Säureadditionssalzen,

mit Ausnahme der Verbindungen der Formel I worin $R^1$ und $R^2$ gleichzeitig Wasserstoff sind, dadurch gekennzeichnet, daß man

a) eine Verbindung der Formel II

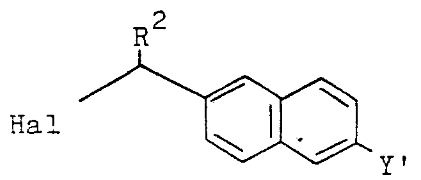

II

worin $R^2$ die zur Formel I angegebene Bedeutung hat, Y' die Reste

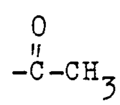

oder $—COOR^7$ bedeutet, wobei $R^7$ $C_1$-$C_4$-Alkyl darstellt und Hal Chlor oder Brom bedeutet, umsetzt mit einem Imidazol der Formel III

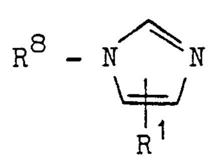

III

worin $R^1$ die zur Formel I angegebene Bedeutung hat und $R^8$ Wasserstoff, niederes Alkanoyl oder Benzoyl bedeutet, zu einer Verbindung der Formel IV

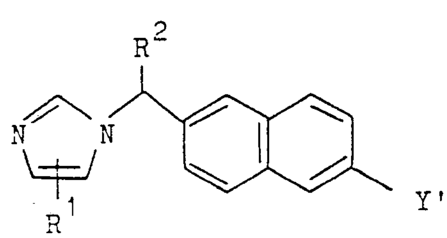

IV

worin $R^1$ und $R^2$ die zur Formel I und Y' die zur Formel II angegebene Bedeutungen haben,

b) gegebenenfalls eine Verbindung der Formel IV, worin Y' den Rest

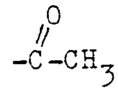

darstellt, oxidiert zu einer Verbindung der Formel I worin Y den Rest —COOH bedeutet,

c) gegebenenfalls eine Verbindung der Formel I worin Y den Rest —$COOR^3$ darstellt, wobei $R^3$ die zur Formel I genannten Bedeutungen hat, jedoch nicht Wasserstoff oder ein Kation darstellt, hydrolysiert zu einer Verbindung der Formel I, worin Y den Rest —COOH darstellt,

d) gegebenenfalls eine Verbindung der Formel I worin Y den Rest —COOH darstellt, in die entsprechenden Ester oder Salze der Formel I, worin Y den Rest —$COOR^3$ mit $R^3 \neq$ Wasserstoff bedeutet, überführt und

e) gegebenenfalls eine erhaltene Verbindung der Formel I in die Säureadditionssalze überführt.

**Patentansprüche** (für den Vertragsstaat AT)

1. Verfahren zur Herstellung von Verbindungen der Formel I

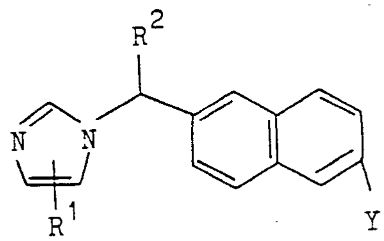

I

worin bedeuten :

$R^1$ Wasserstoff, oder in 2-, 4- oder 5-Position einen $C_1$-$C_4$-Alkylrest

$R^2$ Wasserstoff oder einen $C_1$-$C_4$-Alkylrest

Y einen Rest der Formel —$CO_2R^3$ wobei

$R^3$ Wasserstoff, $C_1$-$C_4$-Alkyl oder ein physiologisch verträgliches Metall-, $NH_4$- oder ein Ammoniumion, das sich von einem primären, sekundären oder tertiären Amin ableitet, oder ein Tetraalkylammoniumion bedeutet.

sowie von deren physiologisch verträglichen Säureadditionssalzen, mit Ausnahme der Verbindungen der Formel I worin $R^1$ und $R^2$ gleichzeitig Wasserstoff sind dadurch gekennzeichnet, daß man

a) eine Verbindung der Formel II

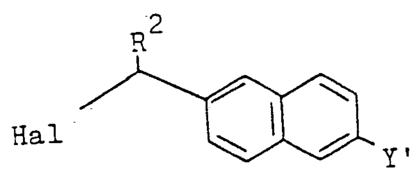

II

worin $R^2$ die zur Formel I angegebene Bedeutung hat, Y' die Reste

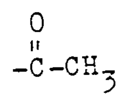

oder —$COOR^7$ bedeutet, wobei $R^7$ $C_1$-$C_4$-Alkyl darstellt und Hal Chlor oder Brom bedeutet, umsetzt mit einem Imidazol der Formel III

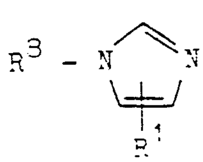

III

worin $R^1$ die zur Formel I angegebene Bedeutung hat und $R^8$ Wasserstoff, niederes Alkanoyl oder Benzoyl bedeutet, zu einer Verbindung der Formel IV

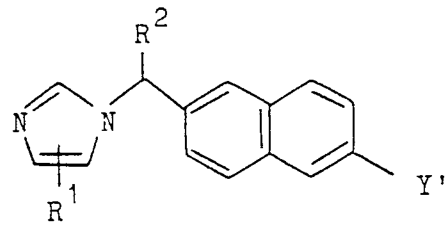

IV

worin $R^1$ und $R^2$ die zur Formel I und Y' die zur Formel II angegebene Bedeutungen haben,

b) gegebenenfalls eine Verbindung der Formel IV, worin Y' den Rest

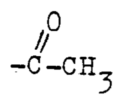

darstellt, oxidiert zu einer Verbindung der Formel I worin Y den Rest —COOH bedeutet,

c) gegebenenfalls eine Verbindung der Formel I worin Y den Rest —$COOR^3$ darstellt, wobei $R^3$ die zur Formel I genannten Bedeutungen hat, jedoch nicht Wasserstoff oder ein Kation darstellt, hydrolysiert zu einer Verbindung der Formel I, worin Y den Rest —COOH darstellt,

d) gegebenenfalls eine Verbindung der Formel I worin Y den Rest —COOH darstellt, in die entsprechenden Ester oder Salze der Formel I, worin Y den Rest —$COOR^3$ mit $R^3 \neq$ Wasserstoff bedeutet, überführt, und

e) gegebenenfalls eine erhaltene Verbindung der Formel I in die Säureadditionssalze überführt.

2. Verfahren gemäß Anspruch 1, dadurch gekennzeichnet, daß man eine Verbindung der Formel I, worin $R^1$ Wasserstoff oder $C_1$-$C_4$-Alkyl in 5-Stellung, $R^2$ Wasserstoff oder $C_1$-$C_2$-Alkyl und Y die —COOH— oder $COOR^3$-Gruppe, worin $R^3$ $C_1$-$C_4$-Alkyl darstellen, bedeuten, oder die physiologisch verträglichen Säureadditionssalze herstellt.

3. Verfahren zur Herstellung eines pharmazeutischen Präparates, dadurch gekennzeichnet, daß man eine Verbindung der Formel I gemäß Anspruch 1 oder ein physiologisch verträgliches Säureadditionssalz dieser Verbindung in eine geeignete Darreichungsform bringt.

**Claims** (for the Contracting States : BE, CH, DE, FR, GB, IT, LI, LU, NL, SE)

1. A compound of the formula I

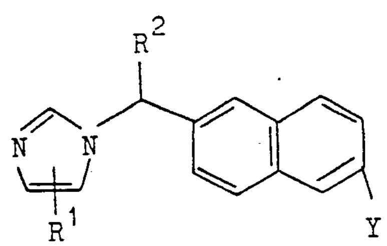

I

in which

$R^1$ denotes hydrogen, or, in the 2-, 4- or 5-position, a $C_1$-$C_4$-alkyl radical,

$R^2$ denotes hydrogen or a $C_1$-$C_4$-alkyl radical,

Y denotes a radical of the formula —$CO_2R^3$, where

$R_3$ denotes hydrogen, $C_1$-$C_4$-alkyl or a physiologically tolerated metal ion, $NH_4$ ion or an ammonium ion which is derived from a primary, secondary or tertiary amine, or a tetraalkylammonium ion,

and the physiologically tolerated acid addition salts with the exception of the compounds of the formula I in which $R^1$ and $R^2$ are both hydrogen.

2. A compound as claimed in claim 1, characterized in that, in formula I, $R^1$ denotes hydrogen or $C_1$-$C_4$-alkyl in the 5-position, $R^2$ denotes hydrogen or $C_1$-$C_2$-alkyl, and Y denotes the —COOH or $COOR^3$ group, in which $R^3$ represents $C^1$-$C^4$-alkyl.

3. A medicament characterized in that it contains a compound as claimed in claim 1 together with pharmaceutically customary auxiliaries and vehicles.

4. A compound as claimed in claim 1 for use as a medicament.

5. A process for the preparation of a compound of the formula I as claimed in claim 1,

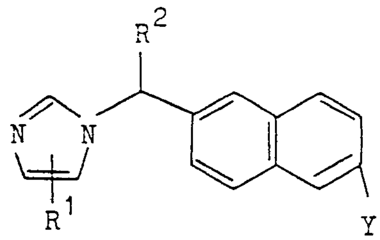

I

in which $R^1$, $R^2$ and Y have the meaning indicated and of the physiologically tolerated acid addition salts thereof, with the exception of the compounds of the formula I in which $R^1$ and $R^2$ are both hydrogen, characterized by

a) reaction of a compound of the formula II

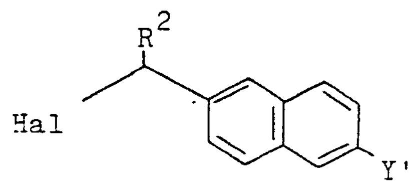

II

in which $R^2$ has the meaning indicated for formula I, Y′ denotes the radicals

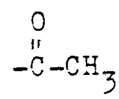

or —$COOR^7$ where $R^7$ represents $C_1$-$C_4$-alkyl, and Hal denotes chlorine or bromine, with an imidazole of the formula III

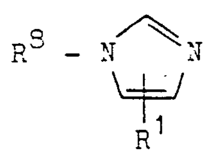

III

in which $R^1$ has the meaning indicated for formula I, and $R^8$ denotes hydrogen, lower alkanoyl or benzoyl, to give a compound of the formula IV

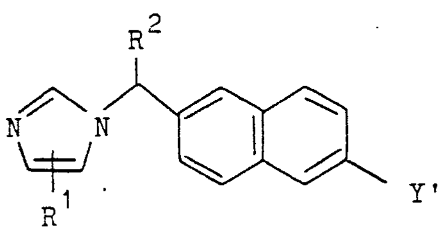

IV

in which $R^1$ and $R^2$ have the meanings indicated for formula I, and Y′ has the meanings indicated for formula II,

b) optionally, oxidation of a compound of the formula IV, in which Y′ represents the radical

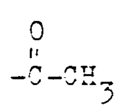

to give a compound of the formula I, in which Y denotes the radical —COOH,

c) optionally, hydrolysis of a compound of the formula I, in which Y represents the radical —$COOR^3$ where $R^3$ has the meanings indicated for formula I, but does not represent hydrogen or a cation, to give a compound of the formula I, in which Y represents the radical —COOH,

d) optionally, conversion of a compound of the formula I, in which Y represents the radical —COOH, into the corresponding esters or salts of the formula I, in which Y denotes the radical —$COOR^3$ with $R^3 \neq$ hydrogen, and

e) optionally, conversion of a resulting compound of the formula I into the acid addition salts.

**Claims** (for the Contracting State AT)

1. A process for the preparation of a compound of the formula I

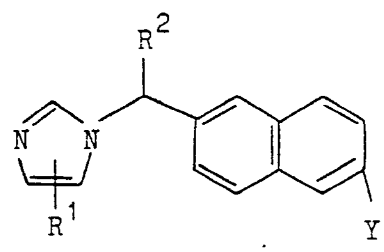

I

in which

$R^1$ denotes hydrogen, or, in the 2-, 4- or 5-position, a $C_1$-$C_4$-alkyl radical,

$R^2$ denotes hydrogen or a $C_1$-$C_4$-alkyl radical

Y denotes a radical of the formula —$CO_2R^3$, where

$R^3$ denotes hydrogen, $C_1$-$C_4$-alkyl or a physiologically tolerated metal ion, $NH_4$ ion or an ammonium ion which is derived from a primary, secondary or tertiary amine, or a tetraalkylammonium ion

and of the physiologically tolerated acid addition salts thereof,

with the exception of the compounds of the formula I in which $R^1$ and $R^2$ are both hydrogen, characterized by

a) reaction of a compound of the formula II

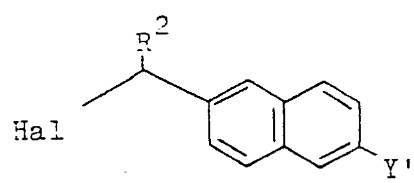

II

in which

$R^2$ has the meaning indicated for formula I,

Y' denotes the radicals

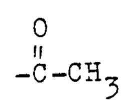

or —$COOR^7$ where $R^7$ represents $C_1$-$C_4$-alkyl, and Hal denotes chlorine or bromine, with an imidazole of the formula III

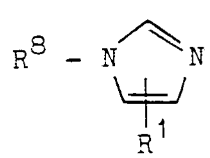

III

in which

$R^1$ has the meaning indicated for formula I, and

$R^8$ denotes hydrogen, lower alkanoyl or benzoyl, to give a compound of the formula IV

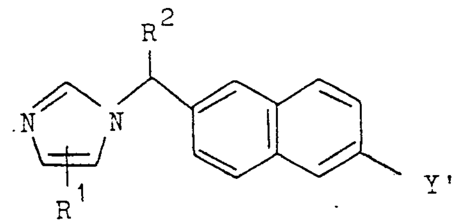

IV

in which

R¹ and R² have the meanings indicated for formula I, and

Y′ has the meanings indicated for formula II,

b) optionally, oxidation of a compound of the formula IV, in which

Y′ represents the radical

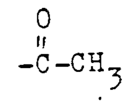

to give a compound of the formula I, in which Y denotes the radical —COOH,

c) optionally, hydrolysis of a compound of the formula I, in which Y represents the radical —$COOR^3$ where $R^3$ has the meanings indicated for formula I, but does not represent hydrogen or a cation, to give a compound of the formula I, in which Y represents the radical —COOH,

d) optionally, conversion of a compound of the formula I, in which Y represents the radical —COOH, into the corresponding esters or salts of the formula I, in which Y denotes the radical —$COOR^3$ with $R^3 \neq$ hydrogen, and

e) optionally, conversion of a resulting compound of the formula I into the acid addition salts.

2. The process as claimed in claim 1, characterized by preparation of a compound of the formula I in which $R^1$ denotes hydrogen or $C_1$-$C_4$-alkyl in the 5-position, $R^2$ denotes hydrogen or $C_1$-$C_2$-alkyl and Y denotes the —COOH or $COOR^3$ group, in which $R^3$ represents $C_1$-$C_4$-alkyl, or of the physiologically tolerated acid addition salts.

3. A process for the preparation of a pharmaceutical product, characterized in that a compound of the formula I as claimed in claim 1, or a physiologically tolerated acid addition salt of this compound is converted into a suitable dosage form.

**Revendications** (pour les Etats contractants : BE, CH, DE, FR, GB, IT, LI, LU, NL, SE)

1. Composés de formule I

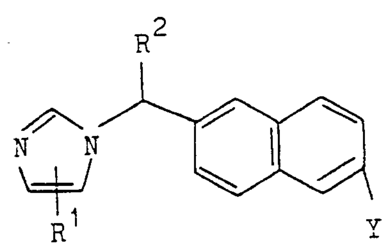

I

dans laquelle :

$R^1$ est hydrogène, ou un reste $C_1$-$C_4$-alkyle en position 2, 4 ou 5

$R^2$ est hydrogène ou un reste $C_1$-$C_4$-alkyle

Y est un reste de formule —$CO_2R^3$, où

$R^3$ est hydrogène, $C_1$-$C_4$-alkyle ou un ion métallique, $NH_4$ ou un ion ammonium dérivé d'une amine primaire, secondaire ou tertiaire, ou un ion tétraalkylammonium physiologiquement compatibles, ainsi que les sels d'addition à des acides physiologiquement compatibles, à l'exception des composés de formule I, dans lesquels $R^1$ et $R^2$ sont simultanément hydrogène.

2. Composés selon la revendication 1, caractérisés en ce que, dans la formule I, $R^1$ est hydrogène ou $C_1$-$C_4$-alkyle en position 5, $R^2$ est hydrogène ou $C_1$-$C_2$-alkyle et Y le groupe COOH ou $COOR^3$, où $R^3$ est un groupe $C_1$-$C_4$-alkyle.

3. Médicaments renfermant un composé selon la revendication 1 ainsi que des adjuvants et vecteurs pharmaceutiques usuels.

4. Composé selon la revendication 1 utilisé comme médicament.

5. Procédé de fabrication de composés de formule I selon la revendication 1,

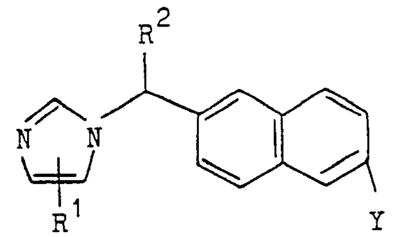

I

dans laquelle $R^1$, $R^2$ et Y ont les significations indiquées, ainsi que leurs sels d'addition à des acides physiologiquement compatibles, à l'exception des composés de formule I, dans lesquels $R^1$ et $R^2$ sont simultanément hydrogène, caractérisé en ce que

a) on fait réagir un composé de formule II

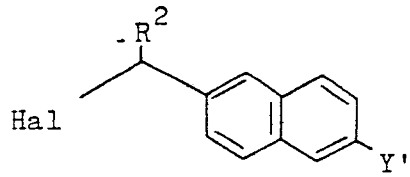

II

dans laquelle $R^2$ a la même signification que pour la formule I, Y' est —CO—$CH_3$ ou —$COOR^7$, $R^7$ représentant un $C_1$-$C_4$-alkyle, et Hal est chlore ou brome, avec un imidazole de formule III

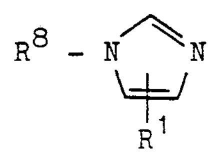

III

dans laquelle $R^1$ a la signification donnée pour la formule I, et $R^3$ est hydrogène, alkanoyle inférieur ou benzoyle, pour obtenir un composé de formule IV

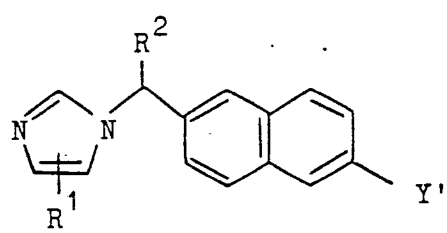

IV

dans laquelle $R^1$ et $R^2$ ont les significations indiquées pour la formule I et Y' celles de la formule II,

b) on oxyde éventuellement un composé de formule IV, dans laquelle Y' représente le reste —CO—$CH_3$, en un composé de formule I dans laquelle Y est —COOH,

c) on hydrolyse éventuellement un composé de formule I, dans laquelle Y est —$COOR^3$ ($R^3$ ayant les mêmes significations que pour la formule I, à l'exception toutefois d'hydrogène ou d'un cation), en un composé de formule I dans laquelle Y est —COOH,

d) on transforme éventuellement un composé de formule I, dans laquelle Y est —COOH, en les esters ou sels correspondants de formule I, dans laquelle Y est —$COOR^3$ avec $R^3 \neq H$, et

e) on transforme éventuellement un composé de formule I en ses sels d'addition à des acides.

**Revendications** (pour l'Etat contractant AT)

1. Procédé de fabrication de composés de formule I

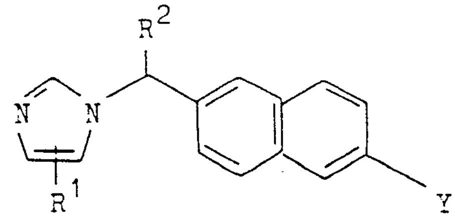

I

dans laquelle :

$R^1$ est hydrogène, ou un reste $C_1$-$C_4$-alkyle en position 2, 4 ou 5

$R^2$ est hydrogène ou un reste $C_1$-$C_4$-alkyle

Y est un reste de formule —$CO_2R^3$, où

$R^3$ est hydrogène, $C_1$-$C_4$-alkyle ou un ion métallique, $NH_4$ ou un ion ammonium dérivé d'une amine primaire, secondaire ou tertiaire, ou un ion tétraalkylammonium physiologiquement compatibles, ainsi que leurs sels d'addition à des acides physiologiquement compatibles, à l'exception des composés de formule I, dans lesquels $R^1$ et $R^2$ sont simultanément hydrogène, caractérisé en ce que

a) on fait réagir un composé de formule II

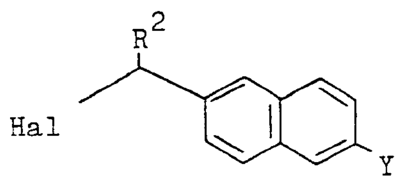

II

dans laquelle $R^2$ a la même signification que pour la formule I, Y' est —CO—$CH_3$ ou —$COOR^7$, $R^7$ représentant un $C_1$-$C_4$-alkyle, et Hal est chlore ou brome, avec un imidazole de formule III

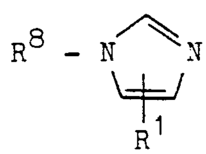

III

dans laquelle $R^1$ a la signification donnée pour la formule I, et $R^3$ est hydrogène, alkanoyle inférieur ou benzoyle, pour obtenir un composé de formule IV

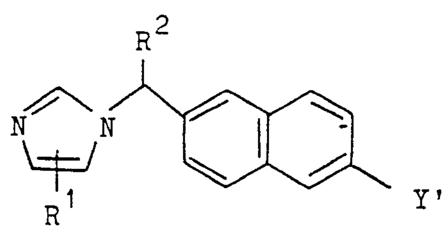

IV

dans laquelle $R^1$ et $R^2$ ont les significations indiquées pour la formule I et Y' celles de la formule II,

b) on oxyde éventuellement un composé de formule IV, dans laquelle Y' représente le reste —CO—$CH_3$, en un composé de formule I dans laquelle Y est —COOH,

c) on hydrolyse éventuellement un composé de formule I, dans laquelle Y est —$COOR^3$ ($R^3$ ayant les mêmes significations que pour la formule I, à l'exception toutefois d'hydrogène ou d'un cation), en un composé de formule I dans laquelle Y est —COOH,

d) on transforme éventuellement un composé de formule I, dans laquelle Y est —COOH, en les esters ou sels correspondants de formule I, dans laquelle Y est —$COOR^3$ avec $R^3 \neq H$, et

e) on transforme éventuellement un composé de formule I en ses sels d'addition d'acides.

2. Procédé selon la revendication 1, caractérisé en ce que l'on fabrique un composé de formule I, dans laquelle $R^1$ est hydrogène ou $C_1$-$C_4$-alkyl en position 5, $R_2$ est hydrogène ou $C_1$-$C_2$-alkyl et Y est le groupe —COOH ou —$COOR^3$, avec $R^3$ = $C_1$-$C_4$-alkyl, ou bien ses sels d'addition d'acides physiologiquement compatibles.

3. Procédé de fabrication d'une préparation pharmaceutique, caractérisé en ce que l'on met un composé de formule I de la revendication 1 ou un sel d'addition d'acide physiologiquement compatible de ce composé sous une forme d'administration appropriée.